# EUROPEAN PATENT APPLICATION

(11) **EP 4 303 207 A1**
(43) Date of publication of application: **10.01.2024**
(21) Application number: 22913660.1
(22) Date of filing: 13.10.2022
(51) Int. Cl.: C07C 29/76, C07C 29/78, C07C 31/18

(54) **REFINEMENT SYSTEM FOR XYLITOL FERMENTED LIQUID, AND METHOD THEREFOR**

(30) Priority: 29.12.2021 CN 202111641269
(71) Applicant: Zhejiang Huakang Pharmaceutical Co., Ltd., Quzhou, Zhejiang 324302 (CN)
(72) Inventor: LI, Mian, Santa Clara, California 95054 (US); ZHEN, Ni, Quzhou, Zhejiang 324302 (CN); LUO, Jiaxing, Quzhou, Zhejiang 324302 (CN); HU, Changhui, Quzhou, Zhejiang 324302 (CN); WU, Qiang, Quzhou, Zhejiang 342302 (CN); YANG, Wulong, Quzhou, Zhejiang 342302 (CN); ZENG, Fangming, Quzhou, Zhejiang 324302 (CN)
(74) Representative: Sun, Yiming
(86) International application number: PCT/CN2022/125221
(87) International publication number: WO 2023/124396

(57) **Abstract**

The present invention relates to a system for a refinement processing of a xylitol fermentation broth, the system includes a fermentation tank, a post-fermentation tank, a ceramic membrane filter, a nanofiltration membrane filter, an activated carbon filter, ion exchange columns, an evaporation tank, a crystallization tank, a centrifuge, and a dryer, which are connected by pipelines. The post-fermentation tank is configured to store the xylitol fermentation broth obtained from the fermentation tank and obtain sediments and supernatant fermentation broth through a standing stratification processing. The ceramic membrane filter, the nanofiltration membrane filter, the activated carbon filter, and the ion exchange columns are configured to perform a filtration and impurity removal processing successively on the supernatant fermentation broth to obtain xylitol ion exchange liquid. The evaporation tank and the crystallization tank are configured to perform an evaporation and crystallization processing on the xylitol ion exchange liquid to obtain xylitol massecuite. The centrifuge is configured to perform a separation processing on the xylitol massecuite to obtain a crystal xylitol and mother liquor respectively. The dryer is configured to perform a drying processing on the crystal xylitol to obtain a refined xylitol crystal product. The present disclosure also discloses a method implemented on the system. The present disclosure significantly reduces an amount of activated carbons, increases a pH value of liquid, and significantly reduces an electrical conductivity and a load of a subsequent ion exchange processing.

## Description

### TECHNICAL FIELD

The present disclosure relates to the technical field of xylitol preparation, in particular, to systems and methods for a refinement processing of xylitol fermentation broth.

### BACKGROUND

Xylitol is a pentose (five-carbon) sugar alcohol. At present, in industrial production, xylitol is commonly produced by the chemical hydrogenation of xylose. Although reaction conditions of biological conversion are mild, there are still some technical bottlenecks. At present, xylitol is prepared by a biological method, which generally takes biomass resources such as corncobs, straws, etc., as raw materials, and obtains hemicellulose hydrolyzate after acid hydrolysis. Since xylose concentration in hydrolyzate is lower after acid hydrolysis, the hydrolyzate cannot be directly used as raw material liquid for biological fermentation. Therefore, hydrolyzate used as the raw material liquid needs to be concentrated, and a commonly used process is evaporation and concentration. During the process mentioned above, sugar, protein, and other substances in the raw material may undergo a Maillard reaction to cause the color of the raw material liquid to be deepened. Fermentation with the raw material prepared by the process also results in a dark color of final fermentation broth, which greatly hinders subsequent refinement processing of the fermentation broth. Patent CN1 08949839A provides a preparation method for a high-purity xylitol. The xylitol fermentation broth is processed through a sterilization, decolorization, ion exchange, concentration, crystallization, and drying processing to obtain a xylitol crystal product. However, an amount of activated carbons in the decolorization process is 20%, which is nearly 100 times the amount in a chemical method, thereby causing a high production cost and producing a large amount of spent activated carbons.

### SUMMARY

### TECHNICAL PROBLEM

The technical problem to be solved by the present disclosure focuses on providing systems and methods for a refinement processing of xylitol fermentation broth. An amount of activated carbons may be significantly reduced while ensuring decolorization and transmittance, and a pH value of material liquid may also be increased, so that an electrical conductivity may be significantly reduced and a load of a subsequent ion exchange processing may also be reduced.

### SOLUTIONS TO THE TECHNICAL PROBLEM

### TECHNICAL SOLUTIONS

The present disclosure is achieved by providing a system for a refinement processing of xylitol fermentation broth. The system includes a fermentation tank, a post-fermentation tank, a ceramic membrane filter, a nanofiltration membrane filter, an activated carbon filter, ion exchange columns, an evaporation tank, a crystallization tank, a centrifuge, and a dryer connected by pipelines in sequence. The post-fermentation tank is configured to store the xylitol fermentation broth obtained from the fermentation tank and perform a standing stratification processing on the xylitol fermentation broth to obtain sediments and supernatant fermentation broth. The ceramic membrane filter, the nanofiltration membrane filter, the activated carbon filter, and the ion exchange columns are configured to perform a filtration and impurity removal processing on the supernatant fermentation broth in sequence to obtain xylitol ion exchange liquid. The evaporation tank and the crystallization tank are configured to perform an evaporation and crystallization processing on the xylitol ion exchange liquid to obtain xylitol massecuite. The centrifuge is configured to perform a separation processing on the xylitol massecuite to obtain a crystal xylitol and mother liquor respectively. The dryer is configured to perform a drying processing on the crystal xylitol to obtain a refined xylitol crystal product.

Furthermore, the ion exchange columns include an anion exchange column and a cation exchange column.

The present disclosure provides a method for a refinement processing of xylitol fermentation broth, which is implemented on the system mentioned above, and the method includes the following steps:
Step 1, a corncob is taken as a raw material, and an impurity removal, rinsing, and acidolysis processing are performed on the raw material to obtain fermentation raw material liquid. Xylitol fermentation broth is obtained by performing a fermentation processing using genetic engineering bacteria after performing a concentration processing on the fermentation raw material liquid until a xylose concentration is greater than 500g/L, and a standing stratification processing is performed on the xylitol fermentation broth to obtain sediments and supernatant fermentation broth respectively.
Step 2, a filtration processing is performed on the supernatant fermentation broth through a ceramic membrane filter to obtain ceramic membrane discharge liquid excluding bacteria and large particle impurities. Process parameters include a feed temperature within a range of 36°C~48°C and a filtration pressure within a range of 0.2MPa~0.4MPa.
Step 3, the ceramic membrane discharge liquid is conveyed to a nanofiltration membrane filter for a nanofiltration membrane filtration processing to retain impurity molecules with a molecular weight greater than 400Da to obtain nanofiltration liquid with a transmittance within a range of 20%~40%. Process parameters of the nanofiltration processing include a feed temperature within a range of 36°C~48°C and a filtration pressure within a range of 2.5MPa~3.3MPa.
Step 4, an activated carbon filtration processing is performed on the nanofiltration liquid by using activated carbons and passing through a cation exchange column and an anion exchange column in sequence for an ion exchange processing to obtain xylitol ion exchange liquid. An amount of the activated carbons is within a range of 0.5%-1.0%, and an electrical conductivity of the xylitol ion exchange liquid is less than 20µs/cm.
Step 5, an evaporation and concentration processing is performed on the xylitol ion exchange liquid until a refraction is within a range of 78%-82%, a cooling and crystallization processing is performed to obtain xylitol massecuite, a centrifugation processing is performed on the xylitol massecuite to obtain a crystal xylitol and mother liquor, and a drying processing is performed on the crystal xylitol to obtain a refined xylitol crystal product.

### BENEFICIAL EFFECTS OF THE PRESENT DISCLOSURE

### BENEFICIAL EFFECTS

Compared with the prior arts, the systems and the methods for the refinement processing of xylitol fermentation broth in the present disclosure include the following features:
1. After the refinement processing, a transmission of a decolorization stage may reach more than 90%
2. A pH value used to process the material liquid is increased, and an electrical conductivity is significantly reduced by absorbing some ions, thereby reducing the load of the subsequent ion exchange process.
3. A xylitol crystal product prepared by a one-time crystallization processing satisfies a national standard requirement, and the crystal is of high purity and has a regular and smooth surface.

### BRIEF DESCRIPTION OF THE DRAWINGS

### DRAWING DESCRIPTION

FIG. 1 is a schematic diagram illustrating an exemplary system for a refinement processing of xylitol fermentation broth according to a preferred embodiment of the present disclosure;
FIG. 2 is a flowchart illustrating an exemplary system and method for a refinement processing of xylitol fermentation broth of the present disclosure; and
FIG. 3 is an analysis spectrum illustrating an HPLC purity of a xylitol crystal product prepared in Embodiment 1 of the present disclosure.

### OPTIMAL EMBODIMENTS FOR IMPLEMENTING THE PRESENT DISCLOSURE OPTIMAL EMBODIMENTS OF THE PRESENT DISCLOSURE

In order to more clearly illustrate the technical issues, the technical solutions, and the beneficial effects of the embodiments of the present disclosure, a specific implementation of the present disclosure with reference to the drawings and embodiments is introduced hereinafter. It should be understood that the specific embodiments described herein are merely used to explain the present disclosure, which are not intended to limit the scope of the present disclosure.

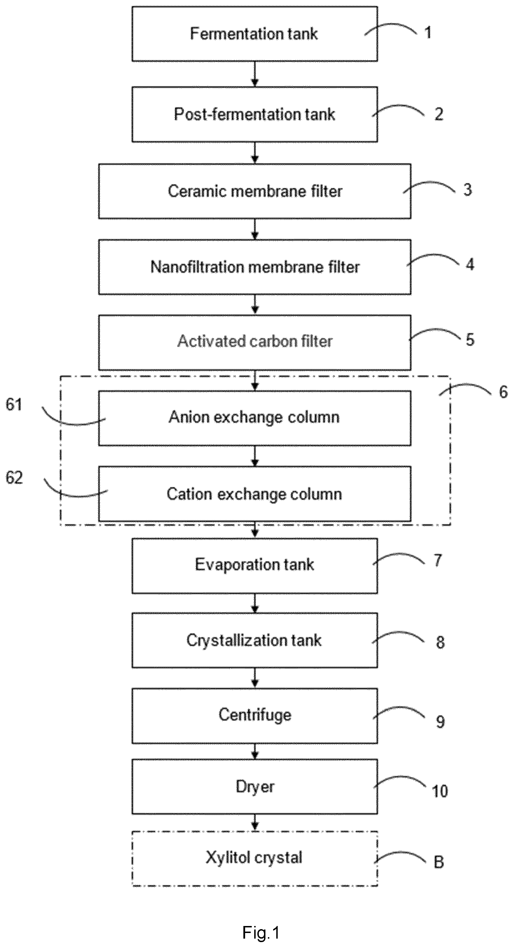

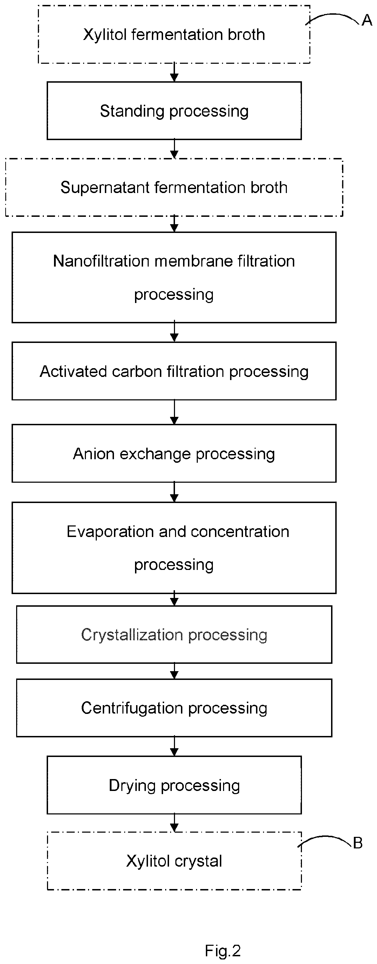

As illustrated in FIG. 1 and FIG. 2, the present disclosure provides a preferred embodiment of a system for a refinement processing of xylitol fermentation broth. The system includes a fermentation tank 1, a post-fermentation tank 2, a ceramic membrane filter 3, a nanofiltration membrane filter 4, an activated carbon filter 5, ion exchange columns 6, an evaporation tank 7, a crystallization tank 8, a centrifuge 9, and a dryer 10 connected by pipelines in sequence. Arrows in FIG.1 illustrate flow directions of materials in the system.

The post-fermentation tank 2 is configured to store xylitol fermentation broth obtained from the fermentation tank 1 and perform a standing stratification processing on the xylitol fermentation broth to obtain sediments and supernatant fermentation broth A. The ceramic membrane filter 3, the nanofiltration membrane filter 4, the activated carbon filter 5, and the ion exchange columns 6 are configured to perform a filtration and impurity removal processing on the supernatant fermentation broth A in sequence to obtain xylitol ion exchange liquid. The evaporation tank 7 and the crystallization tank 8 are configured to perform an evaporation and crystallization processing on the xylitol ion exchange liquid to obtain xylitol massecuite. The centrifuge 9 is configured to perform a separation processing to obtain a crystal xylitol and mother liquor. The dryer 10 is configured to perform a drying processing on the crystal xylitol to obtain a refined xylitol crystal product B.

The ion exchange columns 6 include a cation exchange column 61 and an anion exchange column 62.

As illustrated in FIG. 1 and FIG. 2, the present disclosure also provides a method for a refinement processing of xylitol fermentation broth, which is implemented on the system, and the method includes the following steps:
Step 1, a biomass resource such as a corncob is taken as a raw material, and an impurity removal, rinsing, and acidolysis processing are performed on the raw material to obtain fermentation raw material liquid. The xylitol fermentation broth A is obtained by performing a fermentation processing using genetic engineering bacteria after performing a concentration processing on the fermentation raw material liquid until a xylose concentration is greater than 500g/L, and a standing stratification processing is performed on the xylitol fermentation broth A to obtain sediments and supernatant fermentation broth respectively. The sediments are processed separately.
Step 2, a filtration processing is performed on the supernatant fermentation broth through a ceramic membrane filter to obtain ceramic membrane discharge liquid excluding bacteria and large particle impurities. Process parameters include a feed temperature within a range of 36°C~48°C and a filtration pressure within a range of 0.2MPa~0.4MPa.
Step 3, the ceramic membrane discharge liquid is conveyed to a nanofiltration membrane filter for a nanofiltration membrane filtration processing to retain impurity molecules with a molecular weight greater than 400Da to obtain nanofiltration liquid with a transmittance within a range of 20%~40% Process parameters of the nanofiltration processing include a feed temperature within a range of 36°C~48°C and a filtration pressure within a range of 2.5MPa~3.3MPa.
Step 4, an activated carbon filtration processing is performed on the nanofiltration liquid by using activated carbons. The cation exchange column 61 and the anion exchange column 62 are passed through in sequence for an ion exchange processing to obtain xylitol ion exchange liquid. An amount of the activated carbons is within a range of 0.5%-1.0%, and an electrical conductivity of the xylitol ion exchange liquid is less than 20µs/cm.
Step 5, an evaporation and concentration processing is performed on the xylitol ion exchange liquid until a refraction is within a range of 78%-82%, a cooling and crystallization processing is performed to obtain xylitol massecuite, a centrifugation processing is performed on the xylitol massecuite to obtain a crystal xylitol and mother liquor, and a drying processing is performed on the crystal xylitol to obtain a refined xylitol crystal product B.
Step 6, an evaporation and concentration processing is performed after mixing the mother liquor with the xylitol ion exchange liquid to recycle the mother liquor.

### INVENTION EMBODIMENTS

### EMBODIMENTS OF THE PRESENT DISCLOSUR

The system and the method for the refinement processing of the xylitol fermentation broth of the present disclosure are further described hereinafter with specific embodiments.

### Embodiment 1

A first embodiment of the system and the method for the refinement processing of xylitol fermentation broth of the present disclosure include the following steps:
Step 11, a preparation of xylitol fermentation broth includes taking biomass resources such as a corncob as a raw material, and performing an impurity removal, rinsing, and acidolysis processing on the raw material to obtain fermentation raw material liquid. The fermentation raw material liquid is concentrated until a xylose concentration is more than 500g/L after the impurity removal, decolorization, etc., are performed. The xylitol fermentation broth is obtained through a fermentation processing using genetic engineering bacteria, and a solid content of the xylitol fermentation broth is 11%, an electrical conductivity is 16000 µs/cm, and a pH value is 6.2. In addition to fermentation microorganisms, the xylitol fermentation broth also includes impurities such as large particles of corn steep liquor. A color of the xylitol fermentation broth is dark, and a transmittance is 0%.
Step 12, the xylitol fermentation broth in a volume of 6L is taken and a ceramic membrane filtration processing is performed. A membrane module retains large particle impurities and bacteria with a diameter greater than 50nm. An average flux is 2L/h, a filtration pressure is 0.2MPa, and a material liquid temperature is 45°C during the processing. The electrical conductivity of the ceramic membrane discharge liquid drops to 13460µs/cm and a pH value of the ceramic membrane discharge liquid is 6.4 after the processing.
Step 13, a decolorization processing is performed on the ceramic membrane exchange liquid through the nanofiltration membrane filtration processing. In the processing, a nanofiltration membrane with an interception volume larger than 400 Da is selected. A processing temperature is 45°C, the filtration pressure is 3.3MPa, and the flux is about 6L/h. After the decolorization processing is performed through the nanofiltration membrane filtration processing, the transmittance of the nanofiltration liquid is increased from 0% to 33%, the pH value of the nanofiltration liquid is increased to 6.8, and the electrical conductivity of the nanofiltration liquid is decreased to 7588µs/cm.
Step 14, after the processing of step 13, 0.5% activated carbon powder is added to the system for a secondary decolorization processing through the activated carbon filtration processing. Conditions include performing the decolorization processing at 60°C for 2 hours. Secondary decolorization liquid is activated carbon discharge liquid. The pH value of the activated carbon discharge liquid is 6.9, the electrical conductivity of the activated carbon discharge liquid is dropped to 6865µs/cm, and the transmittance of the activated carbon discharge liquid is 92.4%.
Step 15, after the secondary decolorization liquid is obtained through step 14, an ion exchange processing is performed on the secondary decolorization liquid. A discharge electrical conductivity is controlled to be less than 50 µs/cm during the ion exchange processing, and a manner of water top pressure is adopted to improve the yield of a stage process.
Step 16, an evaporation and concentration processing is performed on the ion exchange liquid until a refraction is 82%, and then a subsequent cooling crystallization processing is performed to obtain a xylitol crystal through a centrifugation and drying processing.

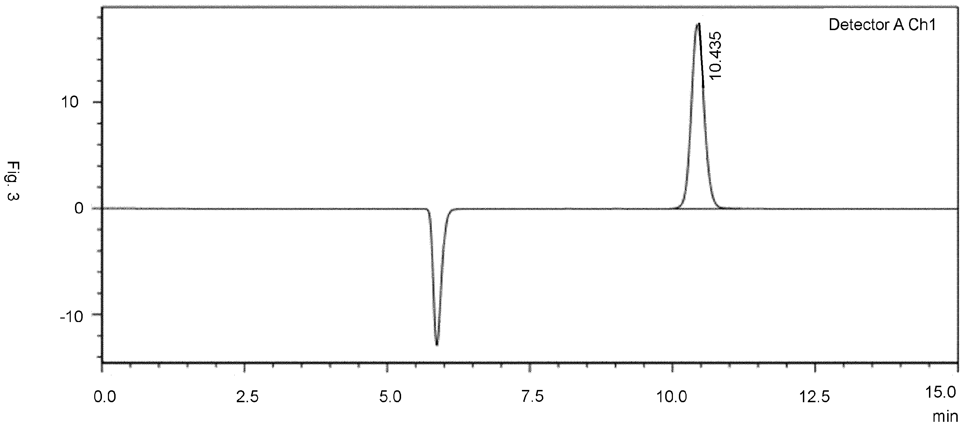

A xylitol crystal obtained through preparation has a white appearance, and a product purity is 99.71% based on a measurement of HPLC. Under an observation through a microscope, a crystal morphology of the xylitol crystal represents as a prismatic shape, a crystal surface is relatively regular and smooth, fine crystals adsorbed on a surface of large particles are less, and other indicators all satisfy a national standard requirement. FIG. 3 is an analysis spectrum illustrating an HPLC purity of a xylitol crystal product prepared in Embodiment 1 of the present disclosure.

Embodiment 2-Exemplary tests of nanofiltration membranes with different interception volumes.

A test manner for selecting a nanofiltration membrane in the decolorization process of the xylitol fermentation broth includes selecting nanofiltration membranes with an interception aperture of 200Da, 400Da, and 600Da to perform a decolorization effect test, and selecting a nanofiltration membrane with a suitable interception aperture by performing a comprehensive evaluation on two aspects of the decolorization effect of the material liquid and an amount of the activated carbons in a subsequent secondary decolorization processing.
(1) The ceramic membrane discharge liquid in a volume of 30L is divided into three equal parts, and each part is in a volume of 10L. A transmittance of the ceramic membrane discharge liquid is 0%, and a pH value of the ceramic membrane discharge liquid is about 6.47.
(2) In a decolorization test for a nanofiltration membrane with an interception aperture of 200Da, the material liquid in a volume of 10L is added to a nanofiltration device, a processing pressure is 3.3MPa, a flux is about 4L/h, and a material liquid temperature is 45°C. The transmittance of the material liquid is about 48% after processed through the nanofiltration membrane. A yield of the process mentioned above is less than 50%. The transmittance of the material liquid may be increased to 96% after processed through the nanofiltration membrane with the interception aperture of 200Da and 0.5% activated carbons are added for the processing.
(3) In a decolorization test for a nanofiltration membrane with an interception aperture of 400Da, the material liquid in a volume of 10L is added to the nanofiltration device, the processing pressure is 3.3MPa, the flux is about 6L/h, and the material liquid temperature is 45°C. The transmittance of the material liquid is about 35% after processed through the nanofiltration membrane. The yield of the process mentioned above is more than 90%. The transmittance of the material liquid may be increased to 92% after processed through the nanofiltration membrane with the interception aperture of 400Da and 0.5% activated carbons are added for the processing.
(4) In a decolorization test for a nanofiltration membrane with an interception aperture of 600Da, the material liquid in a volume of 10L is added to the nanofiltration device, the processing pressure is 3.3MPa, the flux is about 6L/h, and the material liquid temperature is 45°C. The transmittance of the material liquid is about 17.06% after processed through the nanofiltration membrane. The yield of the process mentioned above is more than 90%. The transmittance of the material liquid may be increased to 92% after processed through the nanofiltration membrane with the interception aperture of 600Da and about 2% activated carbons are added for the processing.
(5) Based on a comparison result of decolorization effects of nanofiltration membranes with different interception volumes of 200Da, 400Da, and 600Da, the nanofiltration membrane with an interception aperture of 400Da is selected as the nanofiltration membrane having the best decolorization effect based on an evaluation of the decolorization effect and the amount of the activated carbons in the subsequent secondary decolorization processing comprehensively, which has a high yield and a less amount of the activated carbons in the subsequent secondary decolorization processing.

The embodiments are merely the preferred embodiments of the present disclosure, which are not intended to limit the scope of the present disclosure. Any modifications, equivalent replacements, or improvements made within the spirit and principle of the present disclosure shall be included in the scope of the present disclosure.

## Claims

1. A system for a refinement processing of xylitol fermentation broth, the system including a fermentation tank, a post-fermentation tank, a ceramic membrane filter, a nanofiltration membrane filter, an activated carbon filter, ion exchange columns, an evaporation tank, a crystallization tank, a centrifuge, and a dryer connected by pipelines in sequence, wherein:
the post-fermentation tank is configured to store the xylitol fermentation broth obtained from the fermentation tank and obtain sediments and supernatant fermentation broth through a standing stratification processing;
the ceramic membrane filter, the nanofiltration membrane filter, the activated carbon filter, and the ion exchange columns are configured to perform a filtration and impurity removal processing on the supernatant fermentation broth in sequence to obtain xylitol ion exchange liquid;
the evaporation tank and the crystallization tank are configured to perform an evaporation and crystallization processing on the xylitol ion exchange liquid to obtain xylitol massecuite;
the centrifuge is configured to perform a separation processing on the xylitol massecuite to obtain a crystal xylitol and mother liquor respectively; and
the dryer is configured to perform a drying processing on the crystal xylitol to obtain a refined xylitol crystal product.

2. The system of claim 1, wherein the ion exchange columns include an anion exchange column and a cation exchange column.

3. A method for a refinement processing of xylitol fermentation broth, wherein the method is implemented on a system of claim 1 or claim 2, and the method includes:
step 1, taking a corncob as a raw material, performing an impurity removal, rinsing, and acidolysis processing on the raw material to obtain fermentation raw material liquid, obtaining xylitol fermentation broth by performing a fermentation processing using genetic engineering bacteria after performing a concentration processing on the fermentation raw material liquid until a xylose concentration is greater than 500g/L, and performing a standing stratification processing on the xylitol fermentation broth to obtain sediments and supernatant fermentation broth respectively;
step 2, performing a filtration processing on the supernatant fermentation broth through a ceramic membrane filter to obtain ceramic membrane discharge liquid excluding bacteria and large particle impurities, wherein process parameters include a feed temperature within a range of 36°C~48°C and a filtration pressure within a range of 0.2MPa~0.4MPa;
step 3, conveying the ceramic membrane discharge liquid to a nanofiltration membrane filter for a nanofiltration membrane filtration processing to retain impurity molecules with a molecular weight greater than 400Da to obtain nanofiltration liquid with a transmittance within a range of 20%-40%, wherein process parameters of a nanofiltration processing include a feed temperature within a range of 36°C~48°C and a filtration pressure within a range of 2.5MPa~3.3MPa;
step 4, performing an activated carbon filtration processing on the nanofiltration liquid by using activated carbons and passing through a cation exchange column and an anion exchange column in sequence for an ion exchange processing to obtain xylitol ion exchange liquid, wherein an amount of the activated carbons is within a range of 0.5%~1.0% and an electrical conductivity of the xylitol ion exchange liquid is less than 20µs/cm; and
step 5, performing an evaporation and concentration processing on the xylitol ion exchange liquid until a refraction is within a range of 78%-82%, performing a cooling and crystallization processing to obtain xylitol massecuite, performing a centrifugation processing on the xylitol massecuite to obtain a crystal xylitol and mother liquor, and performing a drying processing on the crystal xylitol to obtain a refined xylitol crystal product.

4. The method of claim 3, further including:
step 6, performing the evaporation and concentration processing after mixing the mother liquor with the xylitol ion exchange liquid to recycle the mother liquor.

5. The method of claim 3, wherein a solid content of the fermentation raw material liquid is within a range of 10%-13%, the electrical conductivity of the fermentation raw material liquid is within a range of 14,000µs/cm~16,000µs/cm, and a pH value of the fermentation raw material liquid is within a range of 6.0-6.8.
